(19) Europäisches Patentamt
European Patent Office
Office européen des brevets

(11) EP 4 253 443 A1

(12) **EUROPEAN PATENT APPLICATION**
published in accordance with Art. 153(4) EPC

(43) Date of publication:
**04.10.2023 Bulletin 2023/40**

(21) Application number: **21898545.5**

(22) Date of filing: **23.11.2021**

(51) International Patent Classification (IPC):
***C08G 18/38*** *(2006.01)* ***C08G 18/70*** *(2006.01)*

(52) Cooperative Patent Classification (CPC):
**C08G 18/38; C08G 18/70**

(86) International application number:
**PCT/KR2021/017236**

(87) International publication number:
**WO 2022/114719 (02.06.2022 Gazette 2022/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.11.2020 KR 20200161937**
**22.11.2021 KR 20210161359**

(71) Applicant: **SKC Co., Ltd.**
**Suwon-si, Gyeonggi-do 16336 (KR)**

(72) Inventors:
- **PAI, Jae Young**
**Suwon-si, Gyeonggi-do 16338 (KR)**
- **KIM, Jeong Moo**
**Suwon-si, Gyeonggi-do 16338 (KR)**
- **HAN, Hyuk Hee**
**Suwon-si, Gyeonggi-do 16338 (KR)**
- **MYUNG, Jung Hwan**
**Suwon-si, Gyeonggi-do 16338 (KR)**
- **YOU, Kyeong Hwan**
**Suwon-si, Gyeonggi-do 16338 (KR)**
- **JUNG, Joo Young**
**Suwon-si, Gyeonggi-do 16338 (KR)**
- **RYU, Ji Yeon**
**Suwon-si, Gyeonggi-do 16338 (KR)**
- **KYUN, Myung Ok**
**Suwon-si, Gyeonggi-do 16338 (KR)**

(74) Representative: **Stolmár & Partner**
**Patentanwälte PartG mbB**
**Blumenstraße 17**
**80331 München (DE)**

# (54) POLYTHIOL COMPOSITION, OPTICAL COMPOSITION, AND OPTICAL PRODUCT

(57) A polythiol composition according to exemplary embodiments includes a main polythiol compound and a sub-compound having a molecular weight higher than that of the main polythiol compound. A peak area (%) at retention times ranging from 34 to 40 minutes in the high performance liquid chromatography (HPLC) analysis graph obtained at a wavelength of 230 nm, which corresponds to the sub-compound, is 2.5% or less. An optical product having improved transmittance and optical properties may be manufactured through fine adjustment of the sub-compound.

EP 4 253 443 A1

[FIG. 1]

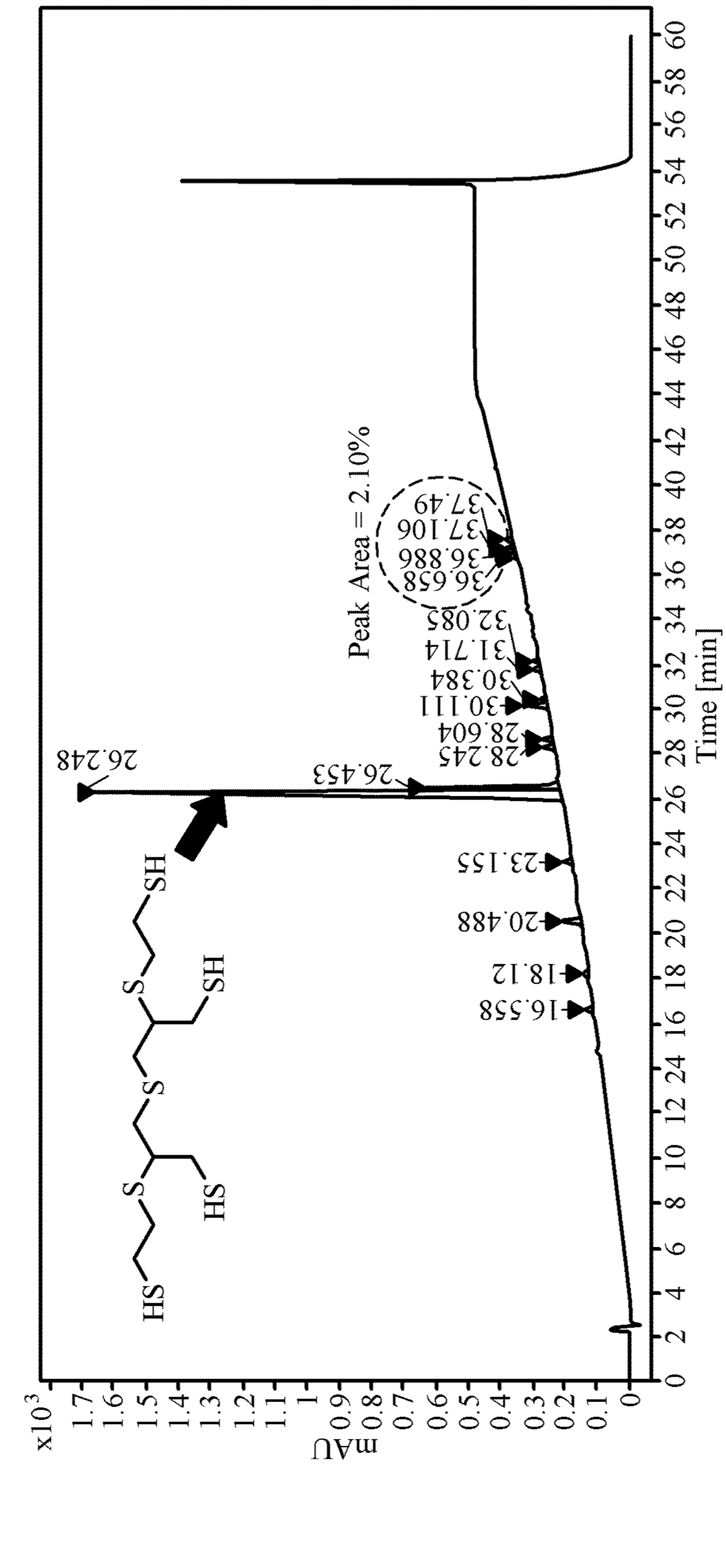
mAU
x10³
Time [min]
Peak Area = 2.10%
26.248
26.453
16.558
18.12
20.488
23.155
28.245
28.604
30.111
30.384
31.714
32.085
36.658
36.886
37.106
37.49

## Description

BACKGROUND

1. Field

The present invention relates to a polythiol composition, an optical composition and an optical product. More particularly, the present inventions relates to a polythiol composition including a polythiol compound and an addition compound, an optical composition including the polythiol compound, and an optical product formed from the optical composition.

2. Description of the Related Art

A polythiol compound is widely used, for example, as a raw material for manufacturing a polyurethane resin. For example, a polythiol compound is used to manufacture an optical lens using a polyurethane resin, and quality such as purity of the polythiol compound as a raw material may directly affect the quality of the optical lens.

For example, a polythiourethane-based compound prepared by reacting a polythiol compound and an isocyanate compound may be used as a base material of the optical lens.

For example, Korean Patent Laid-Open Publication No. 10-1338568 discloses a method for synthesizing a polythiol compound by reacting a polyol compound with thiourea to prepare an isothiouronium salt, and then hydrolyzing it using aqueous ammonia.

Optical properties of an optical product such as a lens may vary depending on a polymerization reaction rate or reactivity of the synthesized polythiol compound and the isocyanate-based compound. For example, the reaction rate may vary by other compounds in addition to the desired target polythiol compound, and thereby reliability of the desired optical properties of the lens may be reduced.

SUMMARY

An object according to exemplary embodiments is to provide a polythiol composition with improved reaction properties and optical properties, and a method for preparation thereof.

An object according to exemplary embodiments is to provide an optical composition including a polythiol composition with improved reaction properties and optical properties.

An object according to exemplary embodiments is to provide an optical product manufactured using the optical composition described above.

To achieve the above objects, according to an aspect of the present invention, there is provided a polythiol composition including: a main polythiol compound; and a sub-compound having a molecular weight higher than that of the main polythiol compound, wherein a peak area (%) at retention times ranging from 34 to 40 minutes in a high performance liquid chromatography (HPLC) analysis graph obtained at a wavelength of 230 nm, which corresponds to the sub-compound, is greater than 0% and 2.5% or less.

In some embodiments, the peak area at the retention times ranging from 34 to 40 minutes in the HPLC analysis graph may be 1.5 to 2.5%.

In some embodiments, a plurality of peaks may be detected at the retention times ranging from 34 to 40 minutes in the HPLC analysis graph, and a sum of areas of the plurality of peaks may be greater than 0% and 2.5% or less.

In some embodiments, the main polythiol compound may include a tetrafunctional polythiol compound corresponding to retention times ranging from 24 to 28 minutes in the HPLC analysis graph.

In some embodiments, the tetrafunctional polythiol compound may include a compound represented by $C_{10}H_{22}S_7$.

In some embodiments, the tetrafunctional polythiol compound may include at least one of compounds represented by Formulas 1-1 to 1-3 below:

[Formula 1-1]

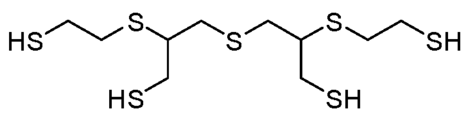

[Formula 1-2]

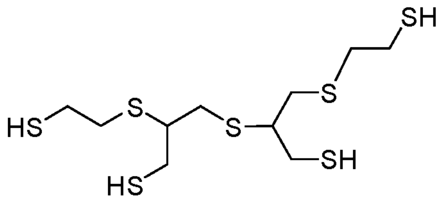

[Formula 1-3]

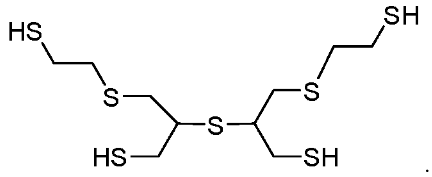

In some embodiments, a peak area (%) at the retention times ranging from 24 to 28 minutes in the HPLC analysis graph may be 80 to 90%.

In some embodiments, the peak area (%) at the retention times ranging from 24 to 28 minutes in the HPLC analysis graph may be 81 to 85%.

In some embodiments, the polythiol composition may have a peak area ratio of 1.5 to 3.1%, which is defined by Formula 1 below:

[Equation 1]

$$\text{Peak area ratio } (\%) = (A/B) \times 100$$

(in Equation 1, A is the peak area (%) included in the retention times ranging from 34 to 40 minutes in the HPLC analysis graph, B is the peak area (%) included in the retention times ranging from 24 to 28 minutes in the HPLC analysis graph).

According to another aspect of the present invention, there is provided an optical composition including: a polythiol composition which includes a main polythiol compound, and a sub-compound having a molecular weight higher than that of the main polythiol compound, wherein a peak area (%) at retention times ranging from 34 to 40 minutes in a high performance liquid chromatography (HPLC) analysis graph obtained at a wavelength of 230 nm, which corresponds to the sub-compound, is greater than 0% and 2.5% or less; and an isocyanate-based compound.

Further, according to another aspect of the present invention, there is provided an optical product including: a copolymer of a polythiol composition and an isocyanate-based compound, wherein the polythiol composition includes a main polythiol compound, and a sub-compound having a molecular weight higher than that of the main polythiol compound, wherein a peak area (%) at retention times ranging from 34 to 40 minutes in a high performance liquid chromatography (HPLC) analysis graph obtained at a wavelength of 230 nm, which corresponds to the sub-compound, is greater than 0% and 2.5% or less.

The polythiol composition according to exemplary embodiments may include a main polythiol compound such as a tetrafunctional polythiol compound and a sub-compound having a molecular weight higher than that of the main polythiol compound. The reactivity of the polythiol composition may be appropriately regulated by finely adjusting the content range of the sub-compound measured by HPLC.

Accordingly, by adjusting the reaction rate of the polythiol composition with the isocyanate-based compound, stria and white turbidity phenomena may be suppressed, and a high transmittance optical lens having a desired refractive index may be obtained with high reliability.

BRIEF DESCRIPTION OF DRAWINGS

FIGS. 1 to 3 are images of high performance liquid chromatography (HPLC) analysis graphs of polythiol compositions prepared according to Examples and Comparative Examples.

DETAILED DESCRIPTION OF THE EMBODIMENTS

Hereinafter, embodiments of the present application will be described in detail. In this regard, the present invention may be altered in various ways and have various embodiments, such that specific embodiments will be illustrated in the drawings and described in detail in the present disclosure. However, the present invention is not limited to the specific embodiments, and it will be understood by those skilled in the art that the present invention is to cover all modifications, equivalents, and alternatives falling within the spirit and scope of the present invention.

Unless otherwise defined, all terms including technical and scientific terms used herein have the same meaning as commonly understood by one of ordinary skill in the art to which the present invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

According to an aspect of the present invention, there is provided a polythiol composition including polythiol compounds.

According to exemplary embodiments, the polythiol composition may include a main polythiol compound and a sub-compound.

The main polythiol compound may refer to a polythiol compound which is a target material included in the polythiol composition and inducing a polymerization reaction with an isocyanate-based compound. For example, the main polythiol compound may be a compound included in the largest content in the polythiol composition. According to exemplary embodiments, the main polythiol compound may be a compound corresponding to a main peak or maximum peak measured through a high performance liquid chromatography (HPLC) analysis graph.

The main polythiol compound may include a trifunctional polythiol compound and/or a tetrafunctional polythiol compound.

As a non-limiting example, the tetrafunctional polythiol compound may include a compound represented by $C_{10}H_{22}S_7$, for example. Non-limiting examples of the tetrafunctional polythiol compound may include compounds represented by Formulas 1-1 to 1-3 below.

[Formula 1-1]

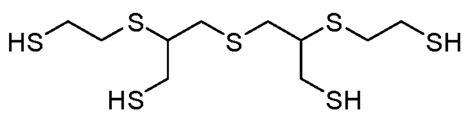

[Formula 1-2]

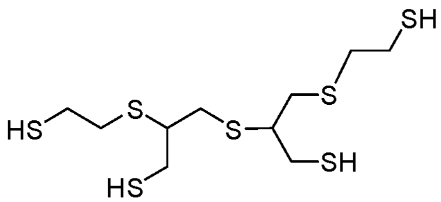

[Formula 1-3]

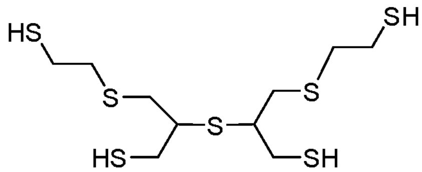

The trifunctional polythiol compound may include a compound represented by $C_7H_{16}S_5$. In one embodiment, the trifunctional polythiol compound may include a compound represented by Formula 2 below.

[Formula 2]

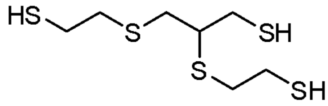

Preferably, the main polythiol compound may include the tetrafunctional polythiol compound. In this case, it may be advantageous in terms of uniformity of optical properties such as a refractive index and mechanical stability such as durability of an optical product such as a lens.

The sub-compound may be a compound having a molecular weight higher than that of the main polythiol compound. For example, the sub-compound may include a reaction initiating material (e.g., 2-mercaptoethanol and epihalohydrin) for synthesizing a polythiol compound, an intermediate such as a polyol compound, or an aggregate or oligomer such as a polythiol compound.

According to exemplary embodiments, the sub-compound may be a compound corresponding to a peak included in retention times ranging from 34 to 40 minutes in a high performance liquid chromatography (HPLC) analysis graph obtained at a wavelength of 230 nm for the polythiol composition.

According to exemplary embodiments, the peak area (%) included in the retention times ranging from 34 to 40 minutes in the HPLC analysis graph corresponding to the sub-compound may be 2.5% or less. For example, in the HPLC analysis graph, the peak area (%) included in the retention times ranging from 34 to 40 minutes may be greater than 0% and 2.5% or less.

In one embodiment, the peak area (%) corresponding to the sub-compound may be in a range of 0.5 to 2.5%, preferably 1 to 2.5%, and more preferably 1.5 to 2.5% or 1.5 to 2.2%.

In some embodiments, a plurality of peaks may be included in the retention times ranging from 34 to 40 minutes in the HPLC analysis graph for the polythiol composition. In this case, the sub-compound may include a plurality of compounds, and a sum of areas of the plurality of peaks within the retention time range may be included within the above-described numerical range.

When the peak area (%) of the sub-compound is excessively increased, a reaction rate or reactivity of the polythiol composition with the isocyanate-based compound may be excessively decreased, and the purity of the polythiol composition may be reduced. Accordingly, white turbidity phenomenon of an optical product such as a lens manufactured using the polythiol composition may be caused.

When the sub-compound is included in an appropriate amount within the above-described numerical range, excessive flowability and an increase in the reaction rate of the composition may be suppressed. Therefore, the stria phenomenon of the optical product manufactured using the polythiol composition may be prevented.

As described above, in the HPLC analysis graph of the polythiol composition, the main polythiol compound may correspond to the maximum peak. In some embodiments, the main polythiol compound may be included within retention times ranging from about 24 to 28 minutes in the HPLC analysis graph.

In some embodiments, the peak area (%) included in the retention times ranging from 24 to 28 minutes in the HPLC analysis graph may be 80 to 90%. In one embodiment, the peak area (%) included in the retention times ranging from 24 to 28 minutes in the HPLC analysis graph may be 81 to 90%, and preferably 81 to 85%, or 82 to 85%.

Within the above range, purity and desired optical properties of the optical composition or optical product may be obtained, and stria due to an excessive increase in the reaction rate may be prevented.

In some embodiments, a plurality of peaks may be included in the retention times ranging from 24 to 28 minutes in the HPLC analysis graph for the polythiol composition. In this case, the sum of areas of the plurality of peaks within the retention time range may be included within the above-described numerical range.

In exemplary embodiments, a peak area ratio of the polythiol composition, which is defined by Equation 1 below, may be 1.5 to 3.1%.

[Equation 1]

$$\text{Peak area ratio (\%)} = (A/B) \times 100$$

In Equation 1, A is the peak area (%) included in the retention times ranging from 34 to 40 minutes in the HPLC analysis graph, and B is the peak area (%) included in the retention times ranging from 24 to 28 minutes in the HPLC analysis graph.

In one embodiment, the peak area ratio may be 1.8 to 3.1%, and preferably 2.0 to 3.1% or 2.0 to 3.0%.

Within the above peak area ratio, sufficient heat resistance and appropriate reaction rate range may be efficiently implemented, without causing a deterioration in the desired refractive index, transparency and purity obtained from the optical product.

The sub-compound has a molecular weight higher than that of the main polythiol compound, and thus may have a longer retention time than that of the main polythiol compound. The sub-compound may be included in the polythiol composition, for example, as a macro-molecule to function as a reaction rate controller of the polythiol composition.

Additionally, as the sub-compound having a high molecular weight is added, a glass transition temperature (Tg) of an optical product may be increased through an increase in an intermolecular attraction and interaction, and heat resistance may also be enhanced.

According to another aspect of the present invention, there is provided a method for preparing the above-described polythiol composition. As described above, the polythiol composition may include the main polythiol compound and the sub-compound.

The method for preparing a polythiol composition according to exemplary embodiments may include the following steps, processes or actions.

The method for preparing a polythiol composition according to exemplary embodiments may include at least one of the steps, processes or actions described as S10, S20, S30 and S40 below. It should be understood that the following terms "S10, S20, S30 and S40" are used to distinguish processes for the convenience of description and are not intended to limit the sequential order thereof. For example, some or all of the processes of S10, S20, S30 and S40 below may be sequentially conducted, and/or may be conducted with altered order according to process conditions.

S10) Reacting 2-mercaptoethanol and epihalohydrin at a first temperature to form a preliminary polyol compound
S20) Introducing a metal sulfide to the preliminary polyol compound and increasing the temperature to a second temperature higher than the first temperature to produce a polyol intermediate
S30) Reacting the polyol intermediate with thiourea under acidic conditions to produce an isothiouronium salt.
S40) Converting the isothiouronium salt into a polythiol compound.

For example, in step S10, the 2-mercaptoethanol and epihalohydrin may be used as reaction initiating materials to allow a reaction according to Scheme 1 below to proceed.

[Scheme 1]

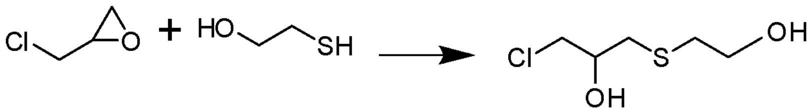

As shown in Scheme 1, epichlorohydrin may be used as the epihalohydrin.

In some embodiments, a basic catalyst may be used in the reaction step of epihalohydrin and 2-mercaptoethanol. For example, in the case of synthesizing a tetrafunctional polythiol compound, examples of the basic catalyst may include tertiary amines such as triethyl amine, quaternary ammonium salts, triphenylphosphine, and trivalent chromium-based compounds. In the case of synthesizing a trifunctional polythiol compound, for example, an alkali metal-containing catalyst such as sodium hydroxide or potassium hydroxide may be used.

According to Scheme 1, a preliminary polyol compound having, for example, the form of a diol compound containing a sulfide bond may be formed.

For example, a content of 2-mercaptoethanol may be 0.5 to 3 moles, preferably 0.7 to 2 moles, and more preferably 0.9 to 1.1 moles based on 1 mole of epihalohydrin. The basic catalyst may be used in an amount of 0.001 to 0.1 moles, 0.005 to 0.03 moles, and preferably 0.007 to 0.015 moles based on 1 mole of epihalohydrin.

Step S10 may be performed, for example, in a state in which a reactor is sufficiently cooled through circulation of a refrigerant in order to suppress an excessive increase in the heat of reaction due to a ring opening reaction of epihalohydrin.

In some embodiments, step S10 may be performed at the first temperature, and the first temperature may be in a range of about 0 to 20 °C, and preferably 15 °C or less, or 5 to 15 °C.

For example, in step S20, a metal sulfide is introduced to the preliminary polyol compound, and the temperature is increased to a second temperature higher than the first temperature to produce a polyol intermediate according to Scheme 2 below.

[Scheme 2]

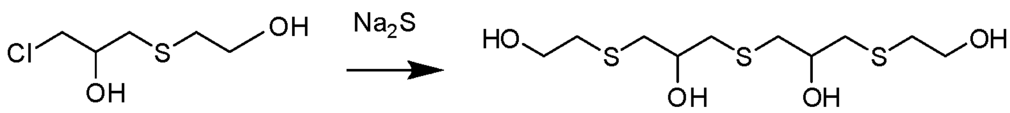


As illustrated in Scheme 2, sulfide bond-containing diol compounds may be further reacted with each other through the metal sulfide to obtain a polyol intermediate including a tetrafunctional polyol compound.

The metal sulfide may include alkali metal sulfide, and as shown in Scheme 2, $Na_2S$ may be used. For example, a polyol intermediate may be formed by introducing an aqueous solution of metal sulfide and stirring a mixture of an aqueous solution of metal sulfide and the preliminary polyol compound.

According to exemplary embodiments, after introducing the metal sulfide, the temperature may be increased to the second temperature while stirring the mixture. The second temperature may be about 40 to 95 °C. Preferably, the second temperature may be greater than 50 °C. In one embodiment, the second temperature may be about 55 to 90 °C, and more preferably 60 to 90 °C.

For example, the metal sulfide may be added dropwise at an intermediate temperature in a range of, for example, 20 °C to 25 °C, which is increased from the first temperature. After completion of the dropping, stirring may be performed by increasing the temperature from the intermediate temperature to the second temperature.

The heat of reaction may be secured through the increased second temperature. Accordingly, an amount of unreacted residual substances generated in step S10 may be appropriately regulated. Thus, the amount of unreacted residual substances may be more effectively controlled or reduced.

For example, the unreacted residual substance in step S10 may generate a large amount of high molecular weight sub-compounds such as oligomers through self-aggregation or self-reaction. However, additional reaction of the unreacted residual substance may be induced by securing sufficient heat of reaction during the production of the polyol intermediate by introducing the metal sulfide, and thereby, the amount of the sub-compound may be appropriately adjusted.

For example, by adjusting the second temperature within the above-described range, the amount of the sub-compound may be maintained so that the peak area (%) at the retention times ranging from 34 to 40 minutes in the HPLC analysis graph is 2.5% or less, preferably in a range of 1.5 to 2.5%, and the above-described peak area ratio may be efficiently secured.

For example, in step S30, the polyol intermediate may be reacted with thiourea. Thus, according to exemplary embodiments, an isothiouronium salt may be obtained.

Reflux under acidic conditions may be used in the production of isothiouronium salts. In order to form the acidic conditions, acidic compounds such as hydrochloric acid, hydrobromic acid, iodic acid, sulfuric acid, phosphoric acid, and the like may be used.

The reflux temperature may be 90 to 120 °C, and preferably 100 to 120 °C, the reflux may be performed for about 1 to 10 hours. As described above, according to exemplary embodiments, as the additional reaction of the additionally added epihalohydrin is performed at the increased second temperature in step S20, the unreacted residual substances may be reduced or maintained in an appropriate amount.

Therefore, excessive generation of by-products is suppressed even under high-temperature reflux conditions, and the reaction rate of the polythiol composition may be appropriately maintained.

For example, the isothiouronium salt may be converted into a polythiol compound in step S40. According to exemplary embodiments, the isothiouronium salt may be hydrolyzed under basic conditions to produce a polythiol compound.

Steps S30 and S40 described above may include thiolation exemplified by Scheme 3 below.

[Scheme 3]

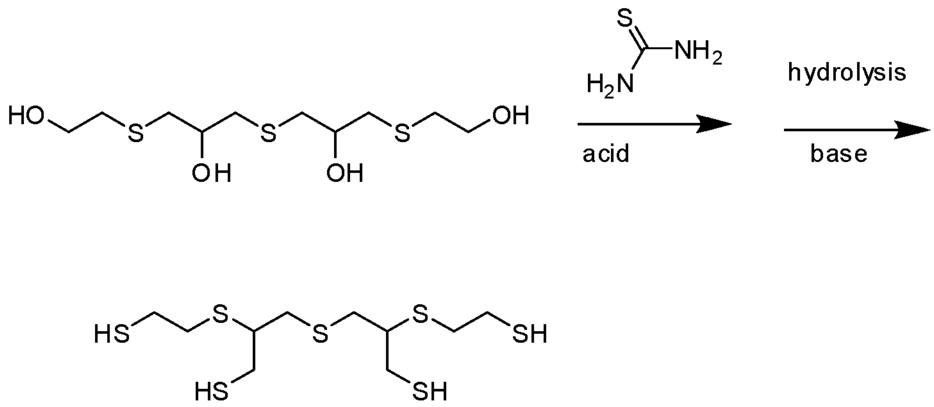


For example, a basic aqueous solution may be added to a reaction solution containing the isothiouronium salt to conduct hydrolysis. The basic aqueous solution may include alkali-metal hydroxide and/or alkaline earth metal hydroxide, such as NaOH, KOH, LiOH, $Ca(OH)_2$, etc.

In one embodiment, the reaction solution containing the isothiouronium salt is cooled to a temperature of 20 to 60 °C, preferably 25 to 55 °C, and more preferably 25 to 50 °C. Thereafter, the basic aqueous solution may be added.

In one embodiment, an organic solvent may be added before adding the basic aqueous solution. An organic solvent having low reactivity or substantially no reactivity and a boiling point exceeding a thiolation reaction temperature may be used so as to allow a thiolation reaction to proceed stably.

Examples of the organic solvent may include toluene, xylene, chlorobenzene, dichlorobenzene and the like. Preferably, toluene may be used in consideration of reaction stability and toxicity from the organic solvent.

The polythiol compound or the polythiol composition obtained as described above may be further purified. For example, by repeatedly performing an acid washing process and a water washing process, impurities included in the polythiol compound may be removed, in addition, the transparency of the optical material prepared from the polythiol composition may be improved. Thereafter, drying, filtration, etc. may be additionally performed.

In one embodiment, an aqueous layer may be separated or removed through layer separation after proceeding with the hydrolysis. The acid washing may be carried out at a temperature of about 20 to 50 □, and preferably about 30 to 40 □ for 20 minutes to 1 hour, or 20 to 40 minutes by introducing an acid solution to the obtained organic phase solution.

After the acid washing, the water washing process may be conducted by adding degassed water having a dissolved oxygen concentration adjusted to 5 ppm or less, preferably 3 ppm or less, and more preferably 2 ppm or less. The water washing process may be conducted at a temperature of about 20 to 50 □, and preferably about 35 to 45 □ for 20 minutes to 1 hour, or 20 to 40 minutes. The water washing process may be repeated two or more times, for example, may be conducted 3 to 6 times.

After the acid washing and the water washing, the residual organic solvent and moisture may be removed by heating under reduced pressure, followed by filtering through a filter to obtain a polythiol compound with high purity.

In some embodiments, a residual moisture content of the polythiol compound or the polythiol composition may be 1,000 ppm or less, preferably in a range of 100 to 500 ppm, and more preferably 150 to 400 ppm.

In some embodiments, the polythiol composition may have a gel permeation chromatography (GPC) purity of 78% or more. For example, the GPC purity of the polythiol composition may be 78 to 85%. Preferably, the GPC purity is 80 to 85%, and more preferably 80 to 84%.

In some embodiments, a liquid refractive index of the polythiol composition at 25 °C may be 1.645 to 1.648, preferably 1.645 to 1.647, and more preferably 1.6450 to 1.6465.

In some embodiments, a thiol value (SHV) of the polythiol composition may be about 96.0 to 98.5 g/eq. Preferably, the SHV is 96.0 to 97.0 g/eq.

The SHV may be measured by dividing the sample weight by the consumed iodine equivalent when titrating a polythiol composition sample using a 0.1N iodine standard solution.

According to the above-described embodiments, the production of the sub-compound may be controlled through the divided introduction of epihalohydrin. However, the present invention is not limited to the above-described preparation method, and the sub-compound may be separately introduced to the polythiol composition in an amount corresponding to the peak area within the above-described range. Additionally, the amount of the sub-compound may be adjusted through other process conditions such as a reaction temperature, and reaction time in addition to the epihalohydrin.

Further, according to another aspect of the present invention, there is provided an optical composition (e.g., a polymerizable composition for an optical material) including the above-described polythiol composition.

The polymerizable composition for an optical material may include the polythiol composition and an isocyanate-based compound. The polythiol composition may include the main polythiol compound and the sub-compound.

The isocyanate-based compound may include a compound that is useable as a monomer for synthesizing polythiourethane. In a preferred embodiment, the isocyanate-based compound may include 1,3-bis(isocyanatomethyl)cyclohexane, hexamethylene diisocyanate, isophorone diisocyanate, xylene diisocyanate, toluene diisocyanate and the like. These may be used alone or in combination of two or more thereof.

The optical composition may further include additives such as a release agent, a reaction catalyst, a thermal stabilizer, an ultraviolet absorber, a bluing agent and the like.

Examples of the release agent may include a fluorine-based nonionic surfactant having a perfluoroalkyl group, a hydroxyalkyl group or a phosphoric acid ester group; a silicone-based nonionic surfactant having a dimethylpolysiloxane group, a hydroxyalkyl group or a phosphoric acid ester group; alkyl quaternary ammonium salts such as trimethylcetyl ammonium salt, trimethylstearyl, dimethylethylcetyl ammonium salt, triethyldodecyl ammonium salt, trioctylmethyl ammonium salt and diethylcyclohexadodecyl ammonium salt; acidic phosphoric acid ester and the like. These may be used alone or in combination of two or more thereof.

As the reaction catalyst, a catalyst used in the polymerization reaction of a polythiourethane resin may be used. For example, dialkyltin halide catalysts, such as dibutyltin dichloride and dimethyltin dichloride; dialkyltin dicarboxylate catalysts such as dimethyltin diacetate, dibutyltin dioctanoate, and dibutyltin dilaurate; dialkyltin dialkoxide catalysts such as dibutyltin dibutoxide and dioctyltin dibutoxide; dialkyltin dithio alkoxide catalysts such as dibutyltin di(thiobutoxide); dialkyltin oxide catalysts such as di(2-ethylhexyl)tin oxide, dioctyltin oxide, and bis(butoxydibutyltin)oxide; dialkyltin sulfide catalysts, and the like may be used. These may be used alone or in combination of two or more thereof.

As examples of the ultraviolet absorber, benzophenone-based, benzotriazole-based, salicylate-based, cyanoacrylate-based, oxanilide-based compounds, and the like may be used. As examples of the thermal stabilizer, metal fatty acid salt-based, phosphorus-based, lead-based, organotin-based compounds, and the like may be used. These may be used alone or in combination of two or more thereof.

The bluing agent may be included as a color regulator of the optical material prepared from the polythiourethane resin. For example, the bluing agent may have an absorption band in a wavelength band from orange to yellow in a visible light region.

Examples of the bluing agent may include a dye, a fluorescent whitening agent, a fluorescent pigment, an inorganic pigment, and the like, and may be appropriately selected according to physical properties or resin color required for the optical product to be manufactured. When a dye is used as the bluing agent, for example, a dye having a maximum absorption wavelength of 520 to 600 nm, and preferably 540 to 580 nm may be used. Preferably, anthraquinone-based dyes may be used.

The polythiourethane resin may be produced through a polymerization reaction of the polythiol compound included in the polythiol composition with the isocyanate-based compound, and the polymerization reaction rate may be adjusted or controlled by the sub-compound included in the polythiol composition.

Accordingly, yellowing or white turbidity phenomenon may be prevented, the generation of stria may be suppressed, and an optical product in which uniform and improved optical properties are maintained for a long period of time may be provided.

In some embodiments, the optical composition may be include about 40 to 60% by weight ("wt.%") of the main polythiol compound, about 40 to 60 wt.% of the isocyanate-based compound, and about 0.01 to 1 wt.% of the additives, based on a total weight of the optical composition.

In some embodiments, the reaction rate of the optical composition included in Equation 1 described below is maintained in a range of 0.15 to 0.30, preferably in a range of 0.15 to 0.25, and more preferably in a range of 0.15 to 0.23 by the sub-compound.

Further, according to another aspect of the present invention, an optical product manufactured through the above-described optical composition may be provided.

For example, after degassing the polymerizable composition under reduced pressure, the resultant composition may be injected into a mold for molding an optical material. Injection into the mold may be performed, for example, in a temperature range of 20 to 40 °C, and preferably 20 to 35 °C.

After injection into the mold, the temperature may be gradually increased, thereby allowing a polymerization reaction of the polythiourethane resin to proceed. The polymerization temperature may be 20 to 150 °C, and preferably 25 to 125 °C. For example, the maximum polymerization temperature may be 100 to 150 °C, preferably 110 to 140 °C, and more preferably 115 to 130 °C.

The heating rate may be 1 to 10 °C/min, preferably 3 to 8 °C/min, and more preferably 4 to 7 °C/min. The polymerization time may be 10 to 20 hours, and preferably 15 to 20 hours.

For example, a lens having uniform optical properties and mechanical properties may be obtained by appropriately controlling the reaction rate within the above temperature range.

After polymerization, the polymerized polythiourethane resin may be separated from the mold to obtain an

optical product. In one embodiment, after separation from the mold, a curing process may be further conducted. The curing process may be conducted in a range of 100 to 150 °C, preferably 110 to 140 °C, and more preferably 115 to 130 °C for about 1 to 10 hours, preferably 2 to 8 hours, and more preferably 3 to 6 hours.

The optical product may be manufactured in the form of a spectacle lens, a camera lens, a light emitting diode, etc. according to a shape of the mold.

The refractive index of the optical product may be adjusted according to the type and/or content ratio of the polythiol compound and the isocyanate-based compound used in the polymerizable composition for an optical material. For example, the refractive index of the optical product may be adjusted in a range of 1.56 to 1.78, 1.58 to 1.76, 1.60 to 1.78, or 1.60 to 1.76, and preferably in a range of 1.65 to 1.75 or 1.69 to 1.75.

In some embodiments, the glass transition temperature (Tg) of the optical product may be 95 to 105 °C, preferably 100 to 105 °C, and more preferably 100 to 104 °C.

The optical product may be improved by further conducting surface treatment such as anti-fouling, color imparting, hard coat, surface polishing, hardness strengthening and the like.

Hereinafter, embodiments provided in the present application will be further described with reference to specific experimental examples. However, the following experimental examples only illustrate the present invention and are not intended to limit the appended claims, and those skilled in the art will obviously understand that various alterations and modifications are possible within the scope and spirit of the present invention. Such alterations and modifications are duly included in the appended claims.

## Example 1

### 1) Synthesis of tetrafunctional polythiol compound

Into a reactor, 60.0 parts by weight ("wt. parts") of water, 0.3 wt. parts of triethylamine, and 73.0 wt. parts of 2-mercaptoethanol (2-ME) were introduced, then cooled to 0 °C, and 88.2 wt. parts of epichlorohydrin (ECH) was slowly added dropwise at a temperature of 15 °C or lower to proceed with a first reaction.

Subsequently, 148.7 wt. parts of a 25% aqueous solution of sodium sulfide ($Na_2S \cdot 9H_2O$) was slowly added dropwise at 25 °C, heated to the second temperature described in Table 1 below, and then stirred for 3 hours. Thereafter, 486.8 wt. parts of 36% hydrochloric acid and 177.8 wt. parts of thiourea were added, and stirred for 3 hours under reflux at 110 °C to proceed with a tiuronium chloride reaction.

After the obtained reaction solution was cooled to 50 °C, 305.6 wt. parts of toluene and 332.6 wt. parts of 50% NaOH were added, and then hydrolysis was conducted at 40 to 60 °C for 3 hours.

Then, the water layer was discarded after performing layer separation for 1 hour, and 120 wt. part of 36% hydrochloric acid was added to the obtained toluene solution, followed by acid washing once at 33 to 40 °C for 30 minutes. After acid washing, 250 wt. parts of degassed water (dissolved oxygen concentration of 2 ppm) was added, and washing was conducted at 35 to 45 °C for 30 minutes. The washing was conducted 4 times. After removing toluene and residual moisture under heating and reduced pressure, it was filtered under reduced pressure through a PTFE type membrane filter thus to obtain 140 wt. parts of polythiol composition including the tetrafunctional polythiol compound represented by the above Formula 1-1 as a main component.

### 2) Preparation of polymerizable composition for an optical material and lens

After uniformly admixing 49.0 wt. parts of the polythiol composition prepared as described above, 51.0 wt. parts of xylene diisocyanate, 0.01 wt. parts of dibutyltin chloride and 0.1 wt. parts of phosphoric acid ester release agent produced by ZELEC® UN Stepan, a defoaming process was conducted at 600 Pa for 1 hour to prepare a polymerizable composition for an optical material.

Then, the composition filtered through a 3 μm Teflon filter was injected into a molding cast provided with a glass mold and a tape. A temperature of the molding cast was slowly increased from 25 to 120 °C at a rate of 5 °C/min, and polymerization was performed at 120 °C for 18 hours. After the polymerization was completed, the molding cast was separated, followed by further curing the product at 120 °C for 4 hours to manufacture a lens sample.

## Examples 2 to 6 and Comparative Examples

Polythiol compositions and lens samples were prepared in the same process as in Example 1, except that the second temperature was changed as described in Table 1 below after adding the sodium sulfide aqueous solution.

In Comparative Example 1, a polythiol composition and a lens sample were prepared in the same manner as in Example 1, except that the aqueous solution of sodium sulfide was added and then stirred at 25 °C.

## Experimental example

### (1) Content assay through HPLC analysis graph

In the polythiol compositions according to the examples and comparative examples, peak areas (%) of sub-compounds measured at the retention times ranging from 34 to 40 minutes were measured through HPLC analysis performed under the following conditions.

<HPLC analysis condition>

i) Equipment: Agilent 1260 Infinity □
ii) Column: ZORBAX Eclipse Plus C18, 5 μm 4.6 × 250 mm
iii) Mobile phase gradient: Acetonitrile (0.1% Formic Acid): Water (0.01M Ammonium Formate) = 35-100: 65-0
iv) Solvent: Acetonitrile (0.1% Formic Acid)
v) Wavelength: 230 nm
vi) Flow rate: 1.0 ml/min, Injection amount: 20 μl, Sample pretreatment: sample: solvent = 0.1 g: 10 g

FIGS. 1 to 3 are images showing high performance liquid chromatography (HPLC) analysis graphs of polythiol compositions prepared according to the examples and comparative examples.
Specifically, FIGS. 1 and 2 show HPLC analysis graphs of Examples 3 and 4, respectively, while FIG. 3 shows HPLC analysis graphs of Comparative Example 1. In
FIGS. 1 to 3, the main peak corresponding to the main polythiol compound is indicated by an arrow, and the peaks at the retention times ranging from 34 to 40 minutes, which correspond to the sub-compounds, are indicated by a dotted circle.

### (2) Evaluation of thiol value (SHV)

About 0.1 g of the polythiol composition prepared in each of the examples and comparative examples was introduced into a beaker, and 25 mL of chloroform was added, followed by stirring the mixture for 10 minutes. Then, 10 mL of methyl alcohol MeOH was added and stirred again for 10 minutes, and then, the resultant solution was titrated with a 0.1N iodine standard solution, followed by measuring SHV according to Equation 1 below (theoretical value: 91.7).

[Equation 1] $\text{SHV (g/eq.)} = \text{Sample weight (g)}/\{0.1 \times \text{Amount of iodine consumed (L)}\}$

### (3) Liquid refractive index

For the polythiol compositions synthesized in the examples and comparative examples, the refractive index at 25 °C was measured using a liquid refractometer (RA-600 (Kyoto Electronics)).

### (4) GPC purity

For the polythiol compositions synthesized in the examples and comparative examples, the purity was measured through gel chromatography analysis performed under the following conditions using an APC system (Waters).

i) Column: Acquity APC XT Column 45A (4.6*150 mm) × 2
ii) Mobile phase: THF
iii) Flow rate: 0.5 mL/min
iv) Total driving time: 10 minutes
v) Injection volume: 10 μl
vi) Detector: RID 40 °C

**(5) Evaluation of stria**

As described above, a lens sample having a diameter of 75 mm and -4.00D was prepared using the polymerizable composition according to each of the examples and comparative examples. A light from a mercury lamp light source was transmitted through the prepared lens sample, and the transmitted light was projected on a white plate to determine the presence or absence of stria according to the presence or absence of contrast. Standards for evaluation are as follows.

◦: Stria not observed
Δ: Fine partial stria observed
x: Stria clearly observed visually

**(6) Evaluation of white turbidity of the lens**

For the lens samples of the examples and comparative examples prepared as described above, each sample was irradiated from a projector in a dark room, and it was visually confirmed whether the lens had haze or an opaque material.
Standards for evaluation are as follows.

◦: No haze
Δ: Partial haze observed
x: Haze clearly observed as a whole

**(7) Measurement of polymerization reaction rate (reactivity slope)**

Using a non-contact viscometer of EMS-1000 (KEM), the standard viscosity (Standard cps) was first confirmed with a viscosity standard solution (Brookfield, 1000 cps, 25 °C). Thereafter, the viscosity was measured at 10 °C for 24 hours for the polymerizable compositions according to the examples and comparative examples, respectively. Using the measured values, mathematical formulation ("mathematization") was conducted with an X-axis as a time and a Y-axis as a viscosity while converting the Y-axis in a logarithmic scale as shown in Mathematical Equation 1 below, and then the reaction rate was derived therefrom.

[Mathematical Equation 1]

$$Y = a \times \exp(b \times X)$$

In Mathematical Equation 1, 'a' value represents an initial viscosity (cps) while 'b' value represents the reaction rate, the measured value was expressed by rounding to the two decimal places of the measured value.

**(8) Measurement of glass transition temperature (Tg)**

The glass transition temperature (Tg) of the lens samples of the examples and comparative examples was measured using a penetration method (load: 50 g, pin tip diameter: Φ 0.5 mm, and heating rate: 10 °C/min) by using a thermomechanical analyzer (TMA Q400, TA instruments).
Evaluation results are shown together in Tables 1 and 2 below.

[TABLE 1]

| | Highest temperature after adding $Na_2S$ (second temperature ) (☐) | Physical property of polythiol composition | | | | | |
|---|---|---|---|---|---|---|---|
| | | SHV (g/ea) | Liquid refractive index | HPLC peak area % at 230 nm (34-40 minutes)(A) | HPLC peak area % at 230 nm (24-28 minutes)( B) | Peak area ratio (A/B) × 100 (%) | GPC purity (%) |
| Example 1 | 80 | 96.2 | 1.6459 | 1.98 | 84.21 | 2.35 | 83 |
| Example2 | 90 | 96.6 | 1.6462 | 1.85 | 84.45 | 2.19 | 83 |

(continued)

| | Highest temperature after adding $Na_2S$ (second temperature ) (□) | Physical property of polythiol composition | | | | | |
|---|---|---|---|---|---|---|---|
| | | SHV (g/ea) | Liquid refractive index | HPLC peak area % at 230 nm (34-40 minutes)(A) | HPLC peak area % at 230 nm (24-28 minutes)( B) | Peak area ratio (A/B) × 100 (%) | GPC purity (%) |
| Examples | 60 | 96.5 | 1.6459 | 2.10 | 83.79 | 2.51 | 82 |
| Example4 | 55 | 96.9 | 1.6463 | 2.21 | 82.40 | 2.68 | 81 |
| Example 5 | 45 | 97.0 | 1.6463 | 2.50 | 81.20 | 3.08 | 80 |
| Example 6 | 100 | 98.5 | 1.6475 | 1.41 | 80.82 | 1.74 | 78 |
| Comparative Example 1 | 15 | 97.2 | 1.6467 | 3.51 | 79.80 | 4.40 | 78 |
| Comparative Example2 | 25 | 97.3 | 1.6468 | 3.11 | 80.18 | 3.88 | 78 |
| Comparative Example3 | 30 | 97.2 | 1.6470 | 2.58 | 80.97 | 3.19 | 80 |

[TABLE 2]

| | Physical property of lens | | | |
|---|---|---|---|---|
| | Stria | White turbidity | Reaction rate | Tg (□) |
| Example 1 | ○ | ○ | 0.22 | 103 |
| Example2 | ○ | ○ | 0.23 | 103 |
| Examples | ○ | ○ | 0.18 | 104 |
| Example4 | ○ | ○ | 0.16 | 104 |
| Example 5 | ○ | ○ | 0.15 | 105 |
| Example 6 | △ | ○ | 0.26 | 100 |
| Comparative Example 1 | ○ | × | 0.11 | 108 |
| Comparative Example2 | ○ | × | 0.12 | 106 |
| Comparative Example3 | ○ | × | 0.14 | 105 |

Referring to Tables 1 and 2, in the case of examples having a peak area of 2.5% or less detected at the retention times ranging from 34 to 40 minutes in the 230 nm HPLC analysis graph, stria and white turbidity phenomena were not substantially observed while maintaining an appropriate reaction rate.

In the case of the comparative examples having an HPLC peak area exceeding 2.5%, white turbidity phenomenon was observed while the polymerization reaction rate was excessively decreased.

## Claims

**1.** A polythiol composition comprising:

a main polythiol compound; and
a sub-compound having a molecular weight higher than that of the main polythiol compound,
wherein a peak area (%) at retention times ranging from 34 to 40 minutes in a high performance liquid chromatography (HPLC) analysis graph obtained at a wavelength of 230 nm, which corresponds to the sub-compound,

is greater than 0% and 2.5% or less.

2. The polythiol composition according to claim 1, wherein the peak area at the retention times ranging from 34 to 40 minutes in the HPLC analysis graph is 1.5 to 2.5%.

3. The polythiol composition according to claim 1, wherein a plurality of peaks are detected at the retention times ranging from 34 to 40 minutes in the HPLC analysis graph, and
a sum of areas of the plurality of peaks is greater than 0% and 2.5% or less.

4. The polythiol composition according to claim 1, wherein the main polythiol compound includes a tetrafunctional polythiol compound corresponding to retention times ranging from 24 to 28 minutes in the HPLC analysis graph.

5. The polythiol composition according to claim 4, wherein the tetrafunctional polythiol compound includes a compound represented by $C_{10}H_{22}S_7$.

6. The polythiol composition according to claim 5, wherein the tetrafunctional polythiol compound includes at least one of compounds represented by Formulas 1-1 to 1-3 below:

[Formula 1-1]

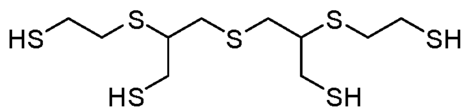

[Formula 1-2]

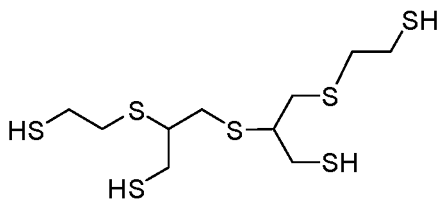

[Formula 1-3]

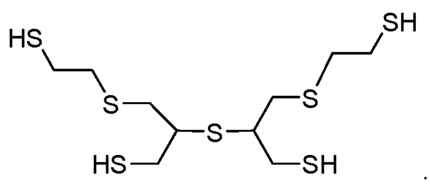

7. The polythiol composition according to claim 1, wherein a peak area (%) at the retention times ranging from 24 to 28 minutes in the HPLC analysis graph is 80 to 90%.

8. The polythiol composition according to claim 1, wherein the peak area (%) at the retention times ranging from 24 to 28 minutes in the HPLC analysis graph is 81 to 85%.

9. The polythiol composition according to claim 1, wherein a peak area ratio defined by Equation 1 below is from 1.5 to 3.1%:

[Equation 1]

$$\text{Peak area ratio (\%)} = (A/B) \times 100$$

(in Equation 1, A is peak area (%) included in the retention times ranging from 34 to 40 minutes in the HPLC analysis graph, B is a peak area (%) included in the retention times ranging from 24 to 28 minutes in the HPLC analysis graph).

**10.** An optical composition comprising:

a polythiol composition which comprises a main polythiol compound, and a sub-compound having a molecular weight higher than that of the main polythiol compound, wherein a peak area (%) at retention times ranging from 34 to 40 minutes in a high performance liquid chromatography (HPLC) analysis graph obtained at a wavelength of 230 nm, which corresponds to the sub-compound, is greater than 0% and 2.5% or less; and
an isocyanate-based compound.

**11.** The optical composition according to claim 10, wherein the peak area at the retention times ranging from 34 to 40 minutes in the HPLC analysis graph is 1.5 to 2.5%.

**12.** The optical composition according to claim 10, wherein the main polythiol compound includes a tetrafunctional polythiol compound corresponding to retention times ranging from 24 to 28 minutes in the HPLC analysis graph.

**13.** The optical composition according to claim 12, wherein the polythiol composition has a peak area ratio of 1.5 to 3.1%, which is defined by Equation 1 below:

[Equation 1]

$$\text{Peak area ratio (\%)} = (A/B) \times 100$$

(in Equation 1, A is the peak area (%) included in the retention times ranging from 34 to 40 minutes in the HPLC analysis graph, B is the peak area (%) included in the retention times ranging from 24 to 28 minutes in the HPLC analysis graph).

**14.** An optical product comprising:

a copolymer of a polythiol composition and an isocyanate-based compound,
wherein the polythiol composition comprises a main polythiol compound, and a sub-compound having a molecular weight higher than that of the main polythiol compound, wherein a peak area (%) at retention times ranging from 34 to 40 minutes in a high performance liquid chromatography (HPLC) analysis graph obtained at a wavelength of 230 nm, which corresponds to the sub-compound, is greater than 0% and 2.5% or less.

**15.** The optical product according to claim 14, a glass transition temperature of the optical product is in a range of 95 to 105 °C.

[FIG. 1]

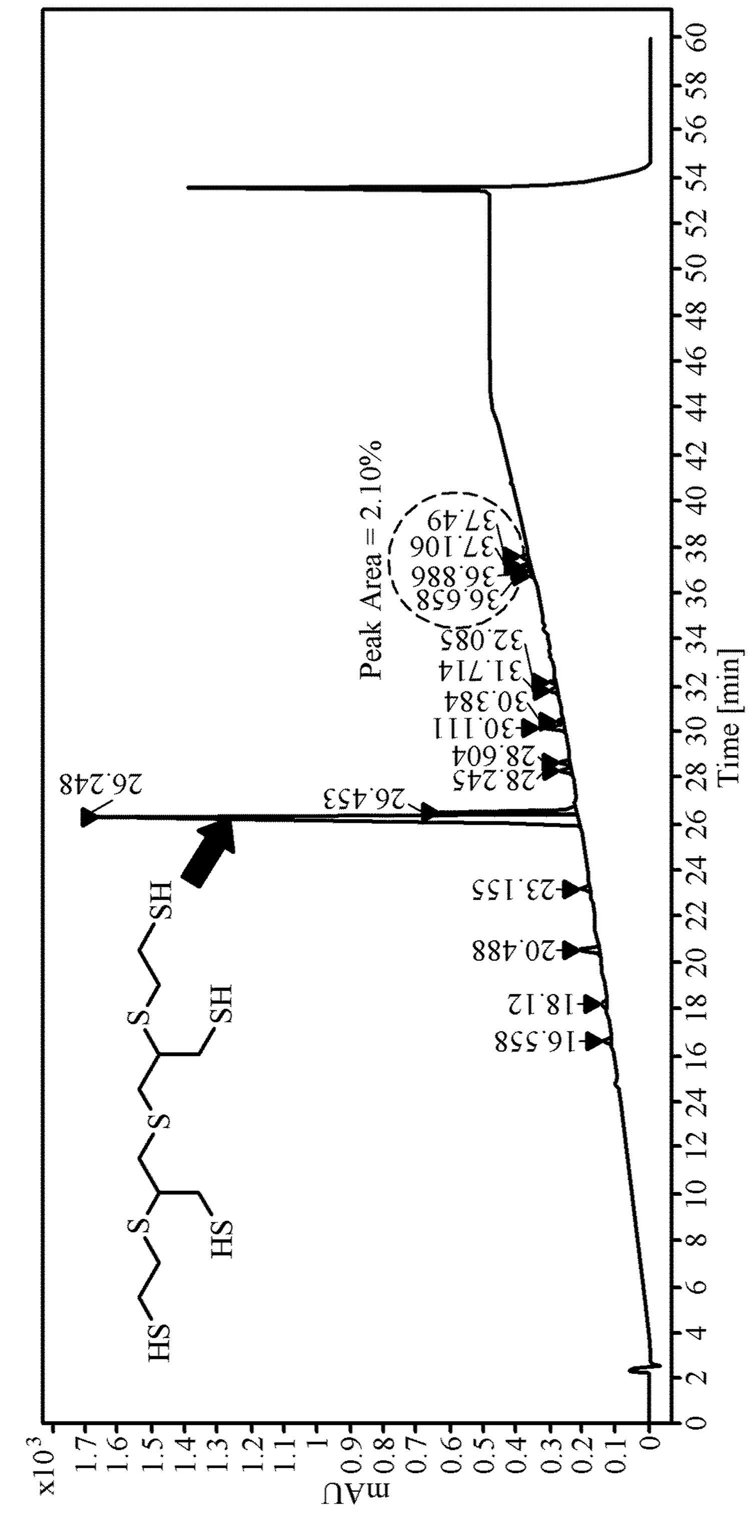
x10[3]
mAU
Time [min]
Peak Area = 2.10%
26.248
26.453
16.558
18.12
20.488
23.155
28.245
28.604
30.111
30.384
31.714
32.085
36.658
36.886
37.106
37.49

[FIG. 2]

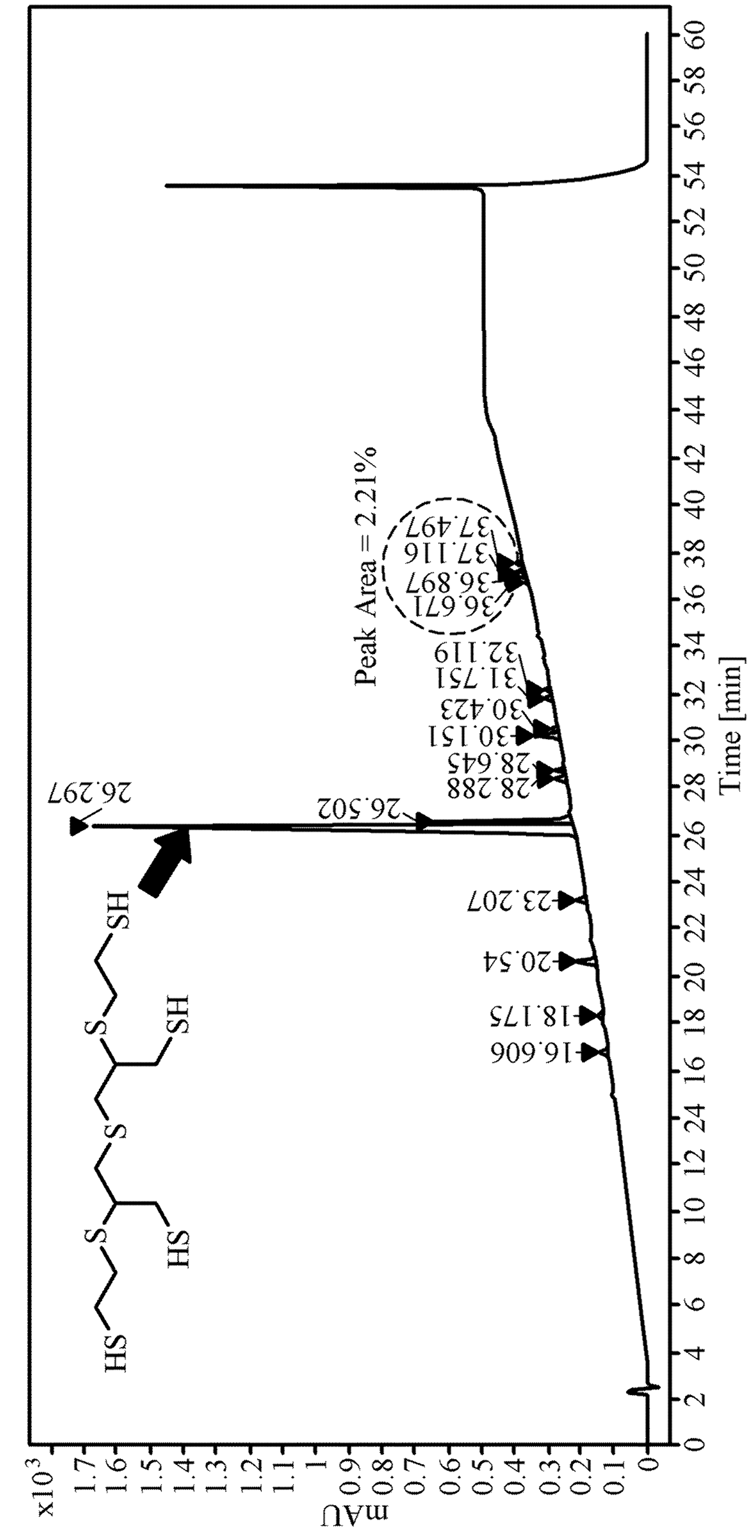
Peak Area = 2.21%
Time [min]
mAU
x10^3
26.297
26.502
16.606
18.175
20.54
23.207
28.288
28.645
30.151
30.423
31.751
32.119
36.671
36.897
37.116
37.497

[FIG. 3]

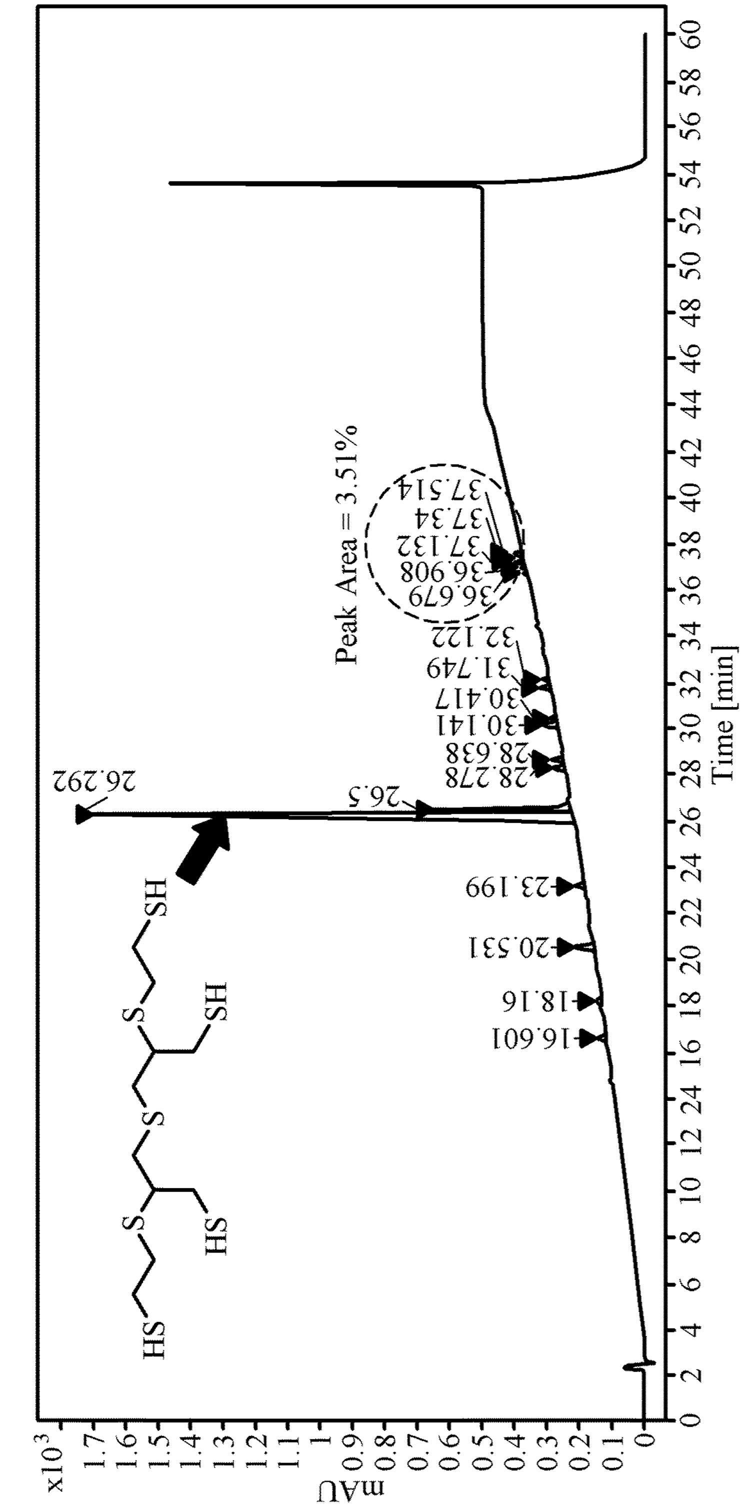
Peak Area = 3.51%
26.292
26.5
28.278
28.638
30.141
30.417
31.749
32.122
36.679
36.908
37.132
37.34
37.514
23.199
20.531
18.16
16.601
Time [min]
mAU
x10^3

**INTERNATIONAL SEARCH REPORT**

International application No.
**PCT/KR2021/017236**

**A. CLASSIFICATION OF SUBJECT MATTER**

**C08G 18/38**(2006.01)i; **C08G 18/70**(2006.01)i

According to International Patent Classification (IPC) or to both national classification and IPC

**B. FIELDS SEARCHED**

Minimum documentation searched (classification system followed by classification symbols)

C08G 18/38(2006.01); B29B 11/00(2006.01); B29D 11/00(2006.01); C07C 319/02(2006.01); C07C 319/20(2006.01); C07C 319/22(2006.01); C07C 321/14(2006.01); C08G 75/02(2006.01); G02B 1/04(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 폴리티올(polythiol), 광학(optical), 고성능 액체크로마토그래피(high-performance liquid chromatography, HPLC), 4관능 폴리티올(tetrafunctional polythiol)

**C. DOCUMENTS CONSIDERED TO BE RELEVANT**

| Category* | Citation of document, with indication, where appropriate, of the relevant passages | Relevant to claim No. |
|---|---|---|
| A | KR 10-2020-0043463 A (HOYA LENS THAILAND LTD.) 27 April 2020 (2020-04-27) See claims 1-7; and example 1. | 1-15 |
| A | KR 10-1894921 B1 (SKC CO., LTD.) 04 September 2018 (2018-09-04) See paragraphs [0014] and [0084]-[0095]. | 1-15 |
| A | KR 10-2122703 B1 (DAEWON F&C INC.) 26 June 2020 (2020-06-26) See paragraphs [0054]-[0055]. | 1-15 |
| A | KR 10-2019-0122729 A (MITSUI CHEMICALS, INC.) 30 October 2019 (2019-10-30) See paragraphs [0195]-[0207]. | 1-15 |
| A | KR 10-2018-0024513 A (SKC CO., LTD.) 08 March 2018 (2018-03-08) See paragraphs [0085]-[0089], [0116] and [0142]. | 1-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 March 2022** | **02 March 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.
**PCT/KR2021/017236**

**C. DOCUMENTS CONSIDERED TO BE RELEVANT**

| Category* | Citation of document, with indication, where appropriate, of the relevant passages | Relevant to claim No. |
|---|---|---|
| A | KR 10-2018-0089363 A (SKC CO., LTD.) 08 August 2018 (2018-08-08) See paragraphs [0088], [0092], [0109] and [0124]. | 1-15 |

Form PCT/ISA/210 (second sheet) (July 2019)

**INTERNATIONAL SEARCH REPORT**
**Information on patent family members**

International application No.
**PCT/KR2021/017236**

| Patent document cited in search report | Publication date (day/month/year) | Patent family member(s) | Publication date (day/month/year) |
|---|---|---|---|
| KR 10-2020-0043463 A | 27 April 2020 | CN 111278806 A | 12 June 2020 |
| | | EP 3660000 A1 | 03 June 2020 |
| | | EP 3660000 A4 | 28 April 2021 |
| | | JP 2019-172630 A | 10 October 2019 |
| | | JP 6797855 B2 | 09 December 2020 |
| | | US 2020-0181075 A1 | 11 June 2020 |
| | | WO 2019-189761 A1 | 03 October 2019 |
| KR 10-1894921 B1 | 04 September 2018 | CN 109627419 A | 16 April 2019 |
| | | CN 109627419 B | 14 April 2020 |
| | | EP 3514187 A1 | 24 July 2019 |
| | | EP 3514187 B1 | 29 July 2020 |
| | | JP 2019-128596 A | 01 August 2019 |
| | | JP 6700444 B2 | 27 May 2020 |
| | | TW 201932449 A | 16 August 2019 |
| | | TW I675821 B | 01 November 2019 |
| | | US 10435512 B2 | 08 October 2019 |
| | | US 2019-0225755 A1 | 25 July 2019 |
| KR 10-2122703 B1 | 26 June 2020 | CN 113784998 A | 10 December 2021 |
| | | WO 2021-206269 A1 | 14 October 2021 |
| KR 10-2019-0122729 A | 30 October 2019 | CN 110446696 A | 12 November 2019 |
| | | EP 3604279 A1 | 05 February 2020 |
| | | EP 3604279 A4 | 23 December 2020 |
| | | JP 6846507 B2 | 24 March 2021 |
| | | KR 10-2236080 B1 | 02 April 2021 |
| | | WO 2018-173820 A1 | 27 September 2018 |
| KR 10-2018-0024513 A | 08 March 2018 | KR 10-1971110 B1 | 22 April 2019 |
| | | TW 201811742 A | 01 April 2018 |
| | | TW I653220 B | 11 March 2019 |
| | | WO 2018-043901 A1 | 08 March 2018 |
| KR 10-2018-0089363 A | 08 August 2018 | KR 10-1885879 B1 | 07 August 2018 |
| | | WO 2018-043896 A1 | 08 March 2018 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 101338568